# EUROPÄISCHE PATENTSCHRIFT

(11) **EP 4 033 244 B1**
(45) Veröffentlichungstag und Bekanntmachung des Hinweises auf die Patenterteilung: **10.09.2025**
(21) Anmeldenummer: 21153216.3
(22) Anmeldetag: 25.01.2021
(51) Int. Cl.: G01N 33/00, G01N 1/22

(54) **GASANALYSEVORRICHTUNG SOWIE VERFAHREN ZUR BESTIMMUNG DER FUNKTIONSTÜCHTIGKEIT EINER GASANALYSEVORRICHTUNG**
GAS ANALYSIS APPARATUS AND METHOD FOR DETERMINING THE FUNCTIONALITY OF A GAS ANALYZER
DISPOSITIF D'ANALYSE DE GAZ, AINSI QUE PROCÉDÉ DE DÉTERMINATION DE L'EFFICACITÉ D'UN DISPOSITIF D'ANALYSE DE GAZ

(43) Veröffentlichungstag der Anmeldung: 27.07.2022
(73) Patentinhaber: M & C TechGroup Germany GmbH, 40885 Ratingen (DE)
(72) Erfinder: RUMM, Hans-Jörg, 46145 Oberhausen (DE)
(74) Vertreter: Berkenbrink, Kai-Oliver

(56) Entgegenhaltungen:
- DE-A1- 102004 031 639
- US-A- 5 376 163
- US-A1- 2016 139 097
- US-A1- 2016 258 916

## Beschreibung

Die Erfindung betrifft eine Gasanalysevorrichtung sowie ein Verfahren zur Bestimmung der Funktionstüchtigkeit einer Gasanalysevorrichtung.

Gasanalysevorrichtungen dienen zur Entnahme von Gas aus einem Gasvolumen sowie zur Analyse des entnommenen Gases. Zur Entnahme des Gases aus einem Gasvolumen können Gasanalysevorrichtungen eine Gasentnahmesonde umfassen. Die Gasentnahmesonde ist derart in ein Gasvolumen einführbar, das Gas aus dem Gasvolumen in die Gasentnahmesonde strömen kann. Das in die Gasentnahmesonde einströmende Gas kann entlang eines Gasströmungsweges der Gasanalysevorrichtung zum Gasanalysator geleitet werden. Der Gasströmungsweg wird durch die Gasentnahmesonde, durch weitere Komponenten der Gasanalysevorrichtung sowie durch diese Komponenten verbindende Gasleitungen definiert.

Das entlang des Gasströmungsweges zum Gasanalysator strömende Gas kann anschließend im Gasanalysator analysiert werden. Bei einem solchen Gasanalysator kann es sich insbesondere um einen Analysator handeln, durch den ein oder mehrere Gase quantitativ und/oder qualitativ analysierbar sind.

Grundsätzlich haben sich entsprechende Gasanalysevorrichtungen zur Gasanalyse bewährt.

Eine Herausforderung stellt jedoch der Einsatz solcher Gasanalysevorrichtungen zur Gasanalyse von staubbehafteten Gasen dar.

Denn durch den Staub des zu analysierenden Gases kann die Funktionstüchtigkeit der Gasanalysevorrichtung derart beeinträchtigt werden, dass diese keine zuverlässige Gasanalyse mehr erlaubt oder auch gar nicht mehr zur Gasanalyse verwendet werden kann. Insbesondere kann der Staub im zu analysierenden Gas den Gasströmungsweg derart zusetzen, dass ein zuverlässiges Leiten des Gases von der Gasentnahmesonde zum Gasanalysator nicht mehr gewährleistet ist.

Von außen ist jedoch regelmäßig nicht erkennbar, ob und inwieweit die Funktionstüchtigkeit einer Gasanalysevorrichtung durch die Anlagerung von Staub beeinträchtigt ist. Es besteht daher das Bedürfnis, eine Technologie zur Verfügung zu stellen, durch die die Funktionstüchtigkeit einer Gasanalysevorrichtung feststellbar ist.

US Patent 5,376,163 offenbart eine bekannte Gasanalysevorrichtung, in welcher das Zusetzen eines Gaseinlassfilters durch einen Durchflusssensor zwischen dem Filter und einer Gaspumpe überwacht wird.

Der Erfindung liegt die Aufgabe zugrunde, eine Gasanalysevorrichtung zur Verfügung zu stellen, durch die die Funktionstüchtigkeit einer Gasanalysevorrichtung bestimmbar ist. Insbesondere soll die Funktionstüchtigkeit einer Gasanalysevorrichtung besonders einfach und zuverlässig bestimmbar sein. Insbesondere soll die Funktionstüchtigkeit einer Gasanalysevorrichtung hinsichtlich ihrer Beeinträchtigung durch Staub einfach und zuverlässig bestimmbar sein.

Ferner liegt der Erfindung die Aufgabe zugrunde, ein Verfahren zur Bestimmung der Funktionstüchtigkeit einer Gasanalysevorrichtung zur Verfügung zu stellen.

Zur Lösung der Aufgabe wird erfindungsgemäß zur Verfügung gestellt eine Gasanalysevorrichtung, welche umfasst:
Eine Gasentnahmesonde zur Entnahme von Gas aus einem Gasvolumen;
einen Gasanalysator zur Analyse von Gas;
einen Gasströmungsweg, entlang dessen ein Gasstrom von der Gasentnahmesonde zum Gasanalysator leitbar ist; und
wenigstens eine Messvorrichtung, durch die der Gasstrom messbar ist;
und eine strömungstechnisch zwischen der Gasentnahmesonde und dem Gasanalysator angeordnete Gaspumpe, durch die der Gasstrom entlang des Gasströmungsweges förderbar ist;
dadurch gekennzeichnet, dass
die wenigstens eine Messvorrichtung wenigstens eine Druckmessvorrichtung umfasst, durch die der Gasdruck im Gasströmungsweg zwischen der Gasentnahmesonde und der Gaspumpe messbar ist und
wenigstens eine Druckmessvorrichtung umfasst, durch die der Gasdruck im Gasströmungsweg zwischen der Gaspumpe und dem Gasanalysator messbar ist.

Die Erfindung beruht insbesondere auf der überraschenden Erkenntnis, dass die Funktionstüchtigkeit einer Gasanalysevorrichtung besonders einfach und zuverlässig dadurch bestimmbar ist, dass die Gasanalysevorrichtung eine Messvorrichtung umfasst, durch die ein entlang des Gasströmungsweges strömender Gasstrom messbar ist. Insoweit liegt der Erfindung insbesondere auch die Erkenntnis zugrunde, dass die Funktionstüchtigkeit der Gasanalysevorrichtung wesentlich durch das Strömungsverhalten des Gasstromes, der entlang des Gasströmungsweges von der Gasentnahmesonde zum Gasanalysator strömt, beeinflusst wird. Wird der Gasströmungsweg insoweit beispielsweise durch Staub derart zugesetzt, dass sich das Strömungsverhalten des Gasstromes entlang des Gasströmungsweges wesentlich verändert, kann die Funktionstüchtigkeit der Gasanalysevorrichtung hierdurch wesentlich beeinträchtigt werden. Beispielsweise wurde erkannt, dass das Strömungsverhalten von Gas entlang des Gasströmungsweges durch Staub dahingehend verändert werden kann, dass ein Druckabfall entlang des Gasströmungsweges, ein reduzierter Gasvolumenstrom entlang des Gasströmungsweges oder ein erhöhter Leitungswiderstand entlang des Gasströmungsweges zu verzeichnen ist. Insoweit wurde erfindungsgemäß erkannt, dass durch Messung dieses Druckabfalls die Funktionstüchtigkeit der Gasanalysevorrichtung bestimmbar ist.

Erfindungsgemäß wurde festgestellt, dass durch eine solche Druckmessvorrichtung die Strömungseigenschaften entlang des Gasströmungsweges besonders einfach und zuverlässig bestimmbar sind. Insbesondere ist durch eine Druckmessvorrichtung einfach und zuverlässig bestimmbar, ob und inwieweit der Gasströmungsweg durch Staub zugesetzt ist.

Die Druckmessvorrichtungen können in Form beliebiger aus dem Stand der Technik bekannter Vorrichtungen zur Messung von Gasdruck vorgesehen sein. Bevorzugt ist ein piezoresistiver Drucksensor als Druckmessvorrichtung vorgesehen.

Die Gasentnahmesonde der erfindungsgemäßen Gasanalysevorrichtung kann grundsätzlich in Form einer beliebigen aus dem Stand der Technik bekannten Gasentnahmesonde zur Entnahme von Gas aus einem Gasvolumen vorliegen. Bevorzugt umfasst die Gasentnahmesonde ein in ein Gasvolumen einführbares Entnahmerohr, das einen Einlass aufweist, über den Gas aus dem Gasvolumen in das Entnahmerohr einleitbar ist. Die Gasentnahmesonde umfasst bevorzugt einen Filter, durch den das aus dem Gasvolumen entnehmbare Gas gefiltert werden kann. Bevorzugt ist ein Keramikfilter oder ein Edelstahlfilter vorgesehen, bevorzugt mit einer Maschenweite von höchstens 3 µm. Durch die Gasentnahmesonde ist ein Abschnitt des Gasströmungsweges der erfindungsgemäßen Gasanalysevorrichtung definiert. Dieser Abschnitt des Gasströmungsweges kann insbesondere vom Einlass des zu entnehmenden Gases in die Gasentnahmesonde durch die Gasentnahmesonde hindurch bis zu einem Auslass der Gasentnahmesonde führen. Soweit die Gasentnahmesonde einen Filter umfasst, führt der Gasströmungsweg durch den Filter hindurch. Sofern die Gasentnahmesonde ferner ein Entnahmerohr umfasst, führt der Gasströmungsweg durch das Entnahmerohr, anschließend durch den Filter und schließlich aus der Gasentnahmesonde heraus.

Da die Strömungseigenschaften des Gasströmungsweges insbesondere durch ein Zusetzen des Filters der Gasentnahmesonde beeinträchtig werden können, kann ein Zusetzen des Gasströmungsweges durch Staub insbesondere, wie oben ausgeführt, durch eine Messung des Gasdrucks stromabwärts der Gasentnahmesonde oder durch Messung des Gasdrucks stromabwärts und stromaufwärts der Gasentnahmesonde zuverlässig bestimmt werden.

Erfindungsgemäß umfasst die erfindungsgemäße Gasanalysevorrichtung eine Gaspumpe. Durch die Gaspumpe ein Abschnitt des Gasströmungsweges definiert. Erfindungsgemäß ist vorgesehen, dass die Gaspumpe strömungstechnisch zwischen der Gasentnahmesonde und dem Gasanalysator angeordnet ist, also die Gaspumpe einen Abschnitt des Gasströmungsweges zwischen der Gasentnahmesonde und dem Gasanalysator bildet. Durch die Gaspumpe kann der Gasstrom entlang des Gasströmungsweges gepumpt beziehungsweise gefördert werden. Die Gaspumpe kann grundsätzlich in Form einer beliebigen aus dem Stand der Technik bekannten Pumpe zur Förderung von Gas vorliegen, beispielsweise in Form einer Membranpumpe oder einer Faltenbalgpumpe.

Erfindungsgemäß ist vorgesehen, dass durch wenigstens eine Druckmessvorrichtung der Druck stromaufwärts der Gaspumpe (also auf dem Abschnitt des Gasströmungsweges zwischen der Gasentnahmesonde und der Gaspumpe) und durch eine Druckmessvorrichtung der Gasdruck stromabwärts der Gaspumpe (also auf dem Abschnitt des Gasströmungsweges zwischen der Gaspumpe und dem Gasanalysator) messbar ist. Hierdurch kann eine Druckdifferenz über die Gaspumpe messbar sein. Erfindungsgemäß wurde festgestellt, dass hierdurch die Strömungseigenschaften eines Gasstromes entlang des Gasströmungsweges zuverlässig und einfach bestimmbar sind.

Nach einer Ausführungsform ist vorgesehen, dass die Gasanalysevorrichtung eine Gaskühler umfasst. Durch den Gaskühler ist ein Abschnitt des Gasströmungsweges definiert. Durch einen Gaskühler ist der entlang des Gasströmungsweges strömende Gasstrom kühlbar. Bevorzugt ist vorgesehen, dass der Gaskühler strömungstechnisch zwischen der Gasentnahmesonde und der Gaspumpe angeordnet ist, also der Gaskühler einen Abschnitt des Gasströmungsweges zwischen der Gasentnahmesonde und der Gaspumpe bildet. Durch eine Kühlung des Gasstromes ist der Taupunkt des Gases absenkbar, um eine Kondensation des Gases im Gasanalysator vermeiden zu können. Grundsätzlich kann ein beliebiger aus dem Stand der Technik bekannter Gaskühler vorliegen, beispielsweise ein Peltier-Gaskühler oder ein Kompressor-Gaskühler.

Erfindungsgemäß wurde festgestellt, dass der Gasdruck des Gasstromes entlang des Gasströmungsweges besonders einfach messbar ist, soweit der Gasdruck stromabwärts des Gaskühlers durch eine Druckmessvorrichtung gemessen wird. Denn aufgrund der geringeren Temperatur des stromabwärts des Gaskühlers gekühlten Gasstromes können Druckmessvorrichtungen verwendet werden, die nur einer geringeren Gastemperatur standhalten müssen.

Nach einer bevorzugten Ausführungsform ist eine der Druckmessvorrichtungen so angeordnet, dass der Gasdruck des Gasstromes im Abschnitt des Gasströmungsweges zwischen dem Gaskühler und der Gaspumpe messbar ist und die andere Druckmessvorrichtung so angeordnet, dass der Gasdruck im Abschnitt des Gasströmungsweges zwischen der Gaspumpe und dem Gasanalysator messbar ist. Denn insoweit sind, wie oben ausgeführt, sowohl die Strömungseigenschaften entlang des Gasströmungsweges besonders zuverlässig durch Messung der Druckdifferenz über die Pumpe und zum anderen gleichzeitig besonders einfach messbar, da das Gas durch den Gaskühler bereits gekühlt ist.

Nach einer Ausführungsform umfasst die Gasanalysevorrichtung eine Heizung, durch die der Gasströmungsweg zumindest abschnittsweise beheizbar ist. Insbesondere kann durch eine solche Heizung der Gasströmungsweg stromaufwärts des Gaskühlers beheizbar sein. Hierdurch kann eine Kondensierung des Gasstromes im Abschnitt des Gasströmungsweges stromaufwärts des Gaskühlers unterdrückt werden. Grundsätzlich kann eine beliebige aus dem Stand der Technik bekannte Heizung zur Beheizung von Gas in Gasanalysevorrichtungen vorgesehen sein, beispielsweise eine elektrische Heizvorrichtung.

Der Gasströmungsweg wird neben der Gasentnahmesonde und etwaigen weiteren Komponenten der Gasanalysevorrichtung (also insbesondere einem Gaskühler und einer Gaspumpe) durch eine oder mehrere Gasleitungen gebildet, die die Gasentnahmesonde (über die etwaig zwischengeschalteten Komponenten) mit dem Gasanalysator verbinden.

Nach einer Ausführungsform umfasst die erfindungsgemäße Gasanalysevorrichtung eine elektronische Datenverarbeitungsvorrichtung (EDV). Die EDV ist bevorzugt zur Verarbeitung der von den Messvorrichtungen gemessenen Daten ausgebildet. Ferner ist die EDV bevorzugt zur Steuerung der Gasanalysevorrichtung ausgebildet. Besonders bevorzugt kann die EDV zur Steuerung der Gasanalysevorrichtung in Abhängigkeit der von den Messvorrichtungen gemessenen und verarbeiteten Daten ausgebildet sein. Nach einer besonders bevorzugten Ausführungsform ist die EDV zur Steuerung der Gasanalysevorrichtung in Abhängigkeit sowohl der von den Messvorrichtungen gemessenen und verarbeiteten Daten als auch von in der EDV gespeicherten Daten ausgebildet. Insoweit kann die EDV insbesondere zur Steuerung der Gasanalysevorrichtung in Abhängigkeit eines Vergleichs der von den Messvorrichtungen gemessenen und verarbeiteten Daten mit den in der EDV gespeicherten Daten ausgebildet sein. Beispielsweise können in der EDV insoweit bestimmte Daten gespeichert sein, wobei die EDV die von den Messvorrichtungen gemessenen und verarbeiteten Daten mit diesen Daten vergleicht und die Gasanalysevorrichtung in Abhängigkeit dieses Vergleichs steuert.

Die EDV kann zum Empfang der von den Messvorrichtungen gemessenen Daten ausgebildet sein. Beispielsweise kann wenigstens eine (kabellose oder kabelgebundene) Datenleitung vorgesehen sein, über die die von den Messvorrichtungen gemessenen Daten an die EDV übertragbar sind. Ferner kann bevorzugt wenigstens eine (kabellose oder kabelgebundene) Signalleitung vorgesehen sein, über die die Gasanalysevorrichtung durch die EDV steuerbar ist, insbesondere durch Abgabe von Steuersignalen über die Signalleitung an die Gasanalysevorrichtung. Nach einer bevorzugten Ausführungsform kann vorgesehen sein, dass eine Gaspumpe der Gasanalysevorrichtung durch die EDV steuerbar ist, insbesondere durch Abgabe von Steuersignalen über die wenigsten seine Signalleitung an die Gaspumpe.

Die EDV umfasst bevorzugt einen Prozessor, bevorzugt in Form eines Microcontrollers. Bevorzugt ist der Prozessor zur Verarbeitung der von den Messvorrichtungen an die EDV übertragenen Daten ausgebildet.

Nach einer bevorzugten Ausführungsform umfasst die EDV einen elektronischen Datenspeicher, der zur Speicherung von Daten ausgebildet ist.

Insbesondere kann, wie oben ausgeführt, vorgesehen sein, dass die EDV beziehungsweise der Prozessor zur Verarbeitung sowohl der von den Messvorrichtungen an die EDV übertragenen Daten als auch den im elektronischen Datenspeicher gespeicherten Daten ausgebildet ist, und die EDV insbesondere zur Steuerung der Gasanalysevorrichtung in Abhängigkeit der von den Messvorrichtungen gemessene und verarbeiteten Daten und den gespeicherten Daten ausgebildet ist.

Beispielsweise können die in der EDV gespeicherten Daten auf empirisch ermittelten Werten, die bei der Messung von Gasströmungen entlang des Gasströmungsweges gemessen worden sind, basieren. Insbesondere können die gespeicherten Daten dabei auf solchen Werten basieren, die eine Funktionsuntüchtigkeit der Gasanalysevorrichtung, insbesondere aufgrund eines Zusetzens des Gasströmungsweges durch Staub, indizieren. Durch einen Vergleich dieser gespeicherten Daten mit den von den Messvorrichtungen gemessenen und durch die EDV verarbeiteten Daten kann die EDV dann eine Funktionsuntüchtigkeit der Gasanalysevorrichtung bestimmen und die Gasanalysevorrichtung in Abhängigkeit von dieser Bestimmung steuern, also beispielsweise durch Abschalten der Gaspumpe steuern.

Gegenstand der Erfindung ist auch ein Verfahren zur Bestimmung der Funktionstüchtigkeit der erfindungsgemäßen Gasanalysevorrichtung, welches die folgenden Schritte umfasst:
Zurverfügungstellung einer hierin beschriebenen Gasanalysevorrichtung;
Messung eines entlang des Gasströmungsweges strömenden Gasstromes;
Bestimmung der Funktionstüchtigkeit der Gasanalysevorrichtung auf Basis der Messergebnisse der Messung.

Dabei kann die Funktionstüchtigkeit der Gasanalysevorrichtung auf Basis eines Vergleichs der Messergebnisse der Messung des Gasstromes mit vorgegebenen Messdaten bestimmt werden. Dieser Vergleich kann, wie oben ausgeführt, durch eine EDV durchgeführt werden.

Ferner kann die Gasanalysevorrichtung auf Basis der Bestimmung der Funktionstüchtigkeit der Gasanalysevorrichtung gesteuert werden. Dabei kann diese Steuerung, wie oben ausgeführt, durch eine EDV erfolgen.

Die Bestimmung der Funktionstüchtigkeit der Gasanalysevorrichtung auf Basis der Messergebnisse der Messung des Gasstromes kann dabei, wie oben ausgeführt, insbesondere mittels der Strömungseigenschaften des Gasstromes entlang des Gasströmungsweges bestimmt werden. Dabei kann die Funktionstüchtigkeit, wie zuvor ausgeführt, insbesondere durch einen Vergleich der durch die Messvorrichtungen gemessenen Messdaten mit vorgegebenen bestimmt werden. Dabei können, wie oben ausgeführt, die durch die Messvorrichtungen gemessenen und an die EDV übertragenen Daten insbesondere durch eine EDV mit in der EDV gespeicherten, vorgegebenen Daten verglichen werden. In Abhängigkeit von diesem Vergleich kann die EDV insoweit beispielsweise die Gasanalysevorrichtung steuern, also beispielsweise eine Abschaltung vornehmen, wenn die Funktionstüchtigkeit der Gasanalysevorrichtung nicht mehr gewährt ist.

Insoweit kann die Funktionstüchtigkeit auf Basis eines Vergleichs der Messergebnisse der Messung des Gasstromes an mehreren Abschnitten des Gasströmungsweges mit vorgegebenen Daten bestimmt werden. Hierzu können die an den Abschnitten gemessenen Messergebnisse beispielsweise in Bezug zueinander gesetzt werden, beispielsweise nach einem vorgegebenen Algorithmus, insbesondere durch die EDV. Beispielsweise kann durch die eine Differenz oder ein Quotient der an den verschiedenen Abschnitten gemessenen Werte der Messergebnisse gebildet werden und der sich danach ergebende Wert mit einem in der EDV gespeicherten Wert verglichen werden.

Alternativ kann die Funktionstüchtigkeit jedoch auch, wie oben ausgeführt, auf Basis eines Vergleichs von Messergebnissen der Messung des Gasstromes an einem Abschnitt des Gasströmungsweges mit vorgegebenen Daten bestimmt werden. Dies kann beispielsweise der Fall sein, wenn für diesen Abschnitt absolute Werte für das Strömungsverhalten des Gasstroms empirisch ermittelt wurden, die eine Funktionsuntüchtigkeit der Gasanalysevorrichtung indizieren.

Auf Basis eines solchen Vergleichs dieser Gasdruckmesswerte kann anschließend die Funktionstüchtigkeit der Gasanalysevorrichtung bestimmt werden.

Nach einer weiteren alternativen Ausführungsform kann die Funktionstüchtigkeit der Gasanalysevorrichtung dadurch bestimmt werden, dass auf Basis der Messergebnisse der Messung des Gasstromes zunächst ein Wert ermittelt wird und auf Basis dieses Wertes die Funktionstüchtigkeit bestimmt wird. Beispielsweise kann der Wert dadurch ermittelt werden, dass auf Basis der Messergebnisse mittels eines vorgegebenen Algorithmus der Wert ermittelt wird. Die Funktionstüchtigkeit kann auf Basis dieses Wertes beispielsweise dadurch bestimmt werden, dass dieser Wert mit einem vorgegebenen Wert verglichen und auf Basis dieses Vergleichs die Funktionstüchtigkeit der Gasanalysevorrichtung bestimmt wird. Der vorgegebene Wert kann insbesondere empirisch ermittelt worden sein und eine Funktionsuntüchtigkeit der Gasanalysevorrichtung indizieren. Der Algorithmus kann insbesondere derart vorgesehen sein, dass durch diesen auf Basis der Messergebnisse ein Wert ermittelt beziehungsweise generiert wird, der mit der Funktionstüchtigkeit der Gasanalysevorrichtung korreliert. Die Ermittlung des Wertes, insbesondere auch mittels des Algorithmus, kann insbesondere durch die EDV erfolgen. Ferner kann auch der Vergleich des ermittelten Wertes mit dem vorgegebenen Wert durch die EDV erfolgen. Durch ein entsprechend ausgebildetes Verfahren lassen sich sehr einfach und zuverlässig Aussagen über die Funktionstüchtigkeit der Gasanalysevorrichtung voraussagen.

Weitere Merkmale der Erfindung ergeben sich aus den Ansprüchen, der Figur und der zugehörigen, nachfolgenden Figurenbeschreibung.

Ein Ausführungsbeispiel der Erfindung wird anhand der beigefügten, sehr schematischen, Figur und der zugehörigen Figurenbeschreibung nachfolgend näher erläutert.

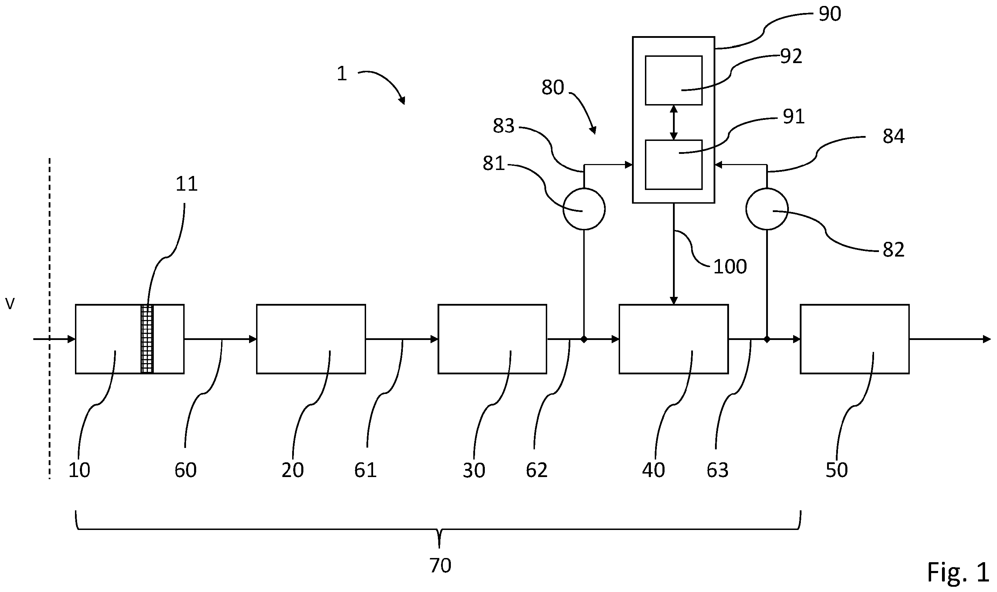

Dabei zeigt
- Figur 1: den schematischen Aufbau eines Ausführungsbeispiels einer erfindungsgemäßen Gasanalysevorrichtung.

In ihrer Gesamtheit ist die Gasanalysevorrichtung in Figur 1 mit dem Bezugszeichen 1 gekennzeichnet.

Die Gasanalysevorrichtung 1 umfasst eine Gasentnahmesonde 10, eine Heizung 20, einen Gaskühler 30, eine Gaspumpe 40 und einen Gasanalysator 50. Dabei sind die Gasentnahmesonde 10, die Heizung 20, der Gaskühler 30, die Gaspumpe 40 und der Gasanalysator 50 über Gasleitungen 60, 61, 62, 63 strömungstechnisch miteinander verbunden. Die Gasentnahmesonde 1, die Heizung 20, der Gaskühler 30, die Gaspumpe 40 sowie die Gasleitungen 60, 61, 62, 63 definieren dabei einen Gasströmungsweg 70, entlang dessen ein Gasstrom von der Gasentnahmesonde 10 zum Gasanalysator 50 leitbar ist. In Strömungsrichtung eines entlang dieses Gasströmungsweges 70 strömenden Gases ist dieser Gasströmungsweg 70 damit gebildet aus dem Gasanalysator 50, der diesem strömungstechnisch nachgeschalteten Gasleitungen 60, der dieser strömungstechnisch nachgeschalteten Heizung 20, der dieser strömungstechnisch nachgeschalteten Gasleitung 61, dem dieser strömungstechnisch nachgeschalteten Gaskühler 30, der diesem strömungstechnisch nachgeschalteten Gasleitung 62, der dieser strömungstechnisch nachgeschalteten Gaspumpe 30 und dem dieser strömungstechnisch nachgeschalteten Gasleitung 63. Durch die Gasleitung 63 ist das entlang des Gasströmungsweges 70 leitbare Gas schließlich in den Gasanalysator 50 leitbar.

Die Gasentnahmesonde 10 weist ein Entnahmerohr auf, durch das Gas aus einem Gasvolumen V entnehmbar und durch einen Edelstahlfilter 11 mit einer Maschenweite von 2 µm leitbar ist.

Das der von der Gasentnahmesonde 10 über die Gasleitung 60 zugeleitete Gas ist durch die Heizung 20 erhitzbar. Hierzu weist die Heizung 20 eine elektrische Heizvorrichtung auf.

Das dem Gaskühler 30 über die Gasleitung 61 von der Heizung 20 zuleitbare Gas ist durch den Gaskühler 30 kühlbar. Der Gaskühler 30 ist dabei als Kompressor-Gaskühler ausgebildet.

Das der Gaspumpe 40 vom Gaskühler 30 über die Gasleitung 62 zuleitbare, gekühlte Gas ist durch die Gaspumpte 40 entlang des Gasströmungsweges 70 leitbar beziehungsweise förderbar. Die Gaspumpe 40 ist als Membranpumpe ausgebildet.

Schließlich ist das durch die Gaspumpe 40 geförderte Gas dem Gasanalysator 50 über die Gasleitung 63 zuführbar.

Der Gasanalysator 50 ist als Multigas-Analysator zur qualitativen und quantitativen Messung von Gasen in Gasgemischen ausgebildet.

Die Gasanalysevorrichtung 1 umfasst ferner eine Messvorrichtung 80, die zwei Druckmessvorrichtungen 81, 82 umfasst. Durch die Messvorrichtung 80 ist der Gasdruck eines entlang des Gasströmungsweges 70 strömenden Gases stromaufwärts und stromabwärts der Gaspumpe 40 messbar. Hierzu umfasst die Messvorrichtung 80 eine erste Druckmessvorrichtung 81, durch die der Gasdruck in der Gasleitung 62 zwischen dem Gaskühler 30 und der Gaspumpe 40 messbar ist. Ferner umfasst die Messvorrichtung 80 eine zweite Druckmessvorrichtung 82, durch die der Gasdruck in der Gasleitung 63 zwischen der Gaspumpe 40 und dem Gasanalysator 50 messbar ist. Bei den Druckmessvorrichtungen 81, 82 handelt es sich um piezoresistive Drucksensoren.

Die Gasanalysevorrichtung 1 umfasst ferner eine Vorrichtung zur elektronischen Datenverarbeitung (EDV), die einen Prozessor 91 und einen Datenspeicher 92 umfasst. Die Druckmessvorrichtungen 81, 82 sind über Datenleitungen 83, 84 derart mit der EDV verbunden, dass die von den Druckmessvorrichtungen 81, 82 gemessenen Messdaten an den Prozessor 91 übertragbar sind. Der Prozessor 91 ist zum Empfang und zur Verarbeitung der über die Datenleitungen 83, 84 übertragenen Messdaten ausgebildet. Der Datenspeicher 92 ist zur Speicherung vorgegebener Daten ausgebildet. Ferner ist die EDV derart ausgebildet, dass im Datenspeicher 92 gespeicherte Daten durch den Prozessor 91 verarbeitbar sind.

Über eine Signalleitung 100 der Gasanalysevorrichtung 1 sind Steuersignale zur Steuerung der Gaspumpe 40 an diese übertragbar.

Die Gasanalysevorrichtung 1 kann beispielsweise wie folgt betrieben und dabei ein Ausführungsbeispiel eines erfindungsgemäßen Verfahrens zur Bestimmung der Funktionstüchtigkeit der Gasanalysevorrichtung 1 durchgeführt werden.

Zunächst wird im Datenspeicher 92 ein vorgegebener Wert gespeichert. Dieser Wert entspricht einem Differenzdruck eines Gasdrucks zwischen der Gasleitung 62 und der Gasleitung 63. Empirisch wurde dabei ermittelt, dass bei Erreichen eines solchen Differenzdrucks über der Gaspumpe 40 die Funktionstüchtigkeit der Gasanalysevorrichtung 1 beeinträchtigt ist. Diese mangelnde Funktionstüchtigkeit der Gasanalysevorrichtung 1 kann insbesondere dadurch gegeben sein, dass die Strömungseigenschaften eines Gasstroms, der entlang des Gasströmungsweges 70 strömt, durch ein Zusetzen des Gasströmungsweges 70 durch Staub beeinträchtigt sind.

Nach Anschalten der Gaspumpe 40 wird Gas aus dem Gasvolumen V über den Einlass des Entnahmerohres der Gasentnahmesonde 10 in die Gasentnahmesonde 10 eingeleitet und anschließend entlang des Gasströmungswegen 70 von der Gasentnahmesonde 10 zum Gasanalysator 50 geleitet und dort analysiert. Während des Transportes von Gas entlang des Gasströmungsweges 70 lagert sich durch das Gas transportierter Staub entlang des Gasströmungsweges 70 ab, insbesondere am Filter der Gasentnahmesonde 10. Hierdurch wird der Gasströmungsweg 70 kontinuierlich mit Staub zugesetzt, so dass sich die Strömungseigenschaften entlang des Gasströmungsweges 70 ändern und die Funktionstüchtigkeit der Gasanalysevorrichtung 1 beeinträchtigen können.

Während dieser Leitung des Gasstromes entlang des Gasströmungsweges 70 wird der Gasdruck in den Gasleitungen 62 und 63 kontinuierlich durch die Druckmessvorrichtungen 81, 82 gemessen und die Messdaten kontinuierlich an den Prozessor 91 der EDV 90 übertragen. Der Prozessor 91 ermittelt auf Basis dieser übertragenen Messdaten kontinuierlich Werte für die Druckdifferenz zwischen den Gasleitungen 62 und 63 und vergleicht diese Wert kontinuierlich mit dem im Datenspeicher 92 gespeicherten Wert, der einer vorgegebenen Druckdifferenz entspricht. Sobald der Prozessor 91 feststellt, dass der ermittelte Wert für eine Druckdifferenz den vorgegebenen Wert erreich hat, hat die EDV 90 eine nicht mehr gegebene Funktionstüchtigkeit der Gasanalysevorrichtung 1 bestimmt. Entsprechend überträgt die EDV 90 dann über die Signalleitung 100 ein Steuersignal zur Abschaltung an die Gaspumpe 40. Nach Abschaltung der Gaspumpe 40 kann die Gasanalysevorrichtung 1 von Staub gereinigt und anschließend die Gaspumpe 40 wieder angeschaltet werden.

## Patentansprüche

1. Gasanalysevorrichtung, welche umfasst:
1.1 eine Gasentnahmesonde (10) zur Entnahme von Gas aus einem Gasvolumen (V);
1.2 einen Gasanalysator (50) zur Analyse von Gas;
1.3 einen Gasströmungsweg (70), entlang dessen ein Gasstrom von der Gasentnahmesonde (10) zum Gasanalysator (50) leitbar ist;
1.4 wenigstens eine Messvorrichtung (80), durch die der Gasstrom messbar ist; und
1.5 eine strömungstechnisch zwischen der Gasentnahmesonde (10) und dem Gasanalysator (50) angeordnete Gaspumpe (40), durch die der Gasstrom entlang des Gasströmungsweges (70) förderbar ist;
**dadurch gekennzeichnet, dass**
1.6 die wenigstens eine Messvorrichtung (80) wenigstens eine Druckmessvorrichtung (81) umfasst, durch die der Gasdruck im Gasströmungsweg (70) zwischen der Gasentnahmesonde (10) und der Gaspumpe (40) messbar ist und wenigstens eine Druckmessvorrichtung (82) umfasst, durch die der Gasdruck im Gasströmungsweg (70) zwischen der Gaspumpe (40) und dem Gasanalysator (50) messbar ist.

2. Gasanalysevorrichtung nach wenigstens einem der vorhergehenden Ansprüche, wobei die Gasentnahmesonde (10) einen Filter (11) umfasst.

3. Gasanalysevorrichtung nach wenigstens einem der vorhergehenden Ansprüche, wobei die Gasanalysevorrichtung (1) einen Gaskühler (30) umfasst.

4. Gasanalysevorrichtung nach wenigstens einem der vorhergehenden Ansprüche, wobei die Gasanalysevorrichtung (1) eine Heizung (20) umfasst.

5. Gasanalysevorrichtung nach den Ansprüchen 3 und 4, wobei die Druckmessvorrichtung (81) so angeordnet ist, dass
der Gasdruck im Gasströmungsweg (70) zwischen dem Gaskühler (30) und der Gaspumpe (40) messbar ist.

6. Verfahren zur Bestimmung der Funktionstüchtigkeit einer Gasanalysevorrichtung, welches die folgenden Schritte umfasst:
9.1 Zur Verfügungstellung einer Gasanalysevorrichtung nach wenigstens einem der vorhergehenden Ansprüche;
9.2 Messung eines entlang des Gasströmungsweges strömenden Gasstromes;
9.3 Bestimmung der Funktionstüchtigkeit der Gasanalysevorrichtung auf Basis der Messergebnisse der Messung.

7. Verfahren nach Anspruch 6, bei dem die Funktionstüchtigkeit der Gasanalysevorrichtung auf Basis eines Vergleichs der Messergebnisse der Messung des Gasstromes mit vorgegebenen Messdaten bestimmt wird.

8. Verfahren nach wenigstens einem der Ansprüche 6 bis 7, bei dem die Gasanalysevorrichtung auf Basis der Bestimmung der Funktionstüchtigkeit der Gasanalysevorrichtung gesteuert wird.

## Claims

1. Gas analysis device, comprising:
1.1 a gas extraction probe (10) for extracting gas from a gas volume (V);
1.2 a gas analyzer (50) for analyzing gas;
1.3 a gas flow path (70) along which a gas flow can be conducted from the gas extraction probe (10) to the gas analyzer (50);
1.4 at least one measuring device (80) by means of which the gas flow can be measured; and
1.5 a gas pump (40) arranged, in terms of the gas flow, between the gas extraction probe (10) and the gas analyzer (50), by means of which gas pump (40) the gas flow can be conveyed along the gas flow path (70);
**characterized in that**
1.6 the at least one measuring device (80) comprises at least one pressure-measuring device (81) by means of which the gas pressure in the gas flow path (70) between the gas extraction probe (10) and the gas pump (40) can be measured and at least one pressure-measuring device (82) by means of which the gas pressure in the gas flow path (70) between the gas pump (40) and the gas analyzer (50) can be measured.

2. Gas analysis device according to at least one of the preceding claims, wherein the gas extraction probe (10) includes a filter (11).

3. Gas analysis device according to at least one of the preceding claims, wherein the gas analysis device (1) includes a gas cooler (30).

4. Gas analysis device according to at least one of the preceding claims, wherein the gas analysis device (1) includes a heater (20).

5. Gas analysis device according to claims 3 and 4, wherein the pressure-measuring device (81) is arranged so that the gas pressure in the gas flow path (70) between the gas cooler (30) and the gas pump (40) can be measured.

6. Method for determining the functional reliability of a gas analysis device, comprising the following steps:
6.1 Providing a gas analysis device according to at least one of the preceding claims;
6.2 Measuring a gas flow flowing along the gas flow path;
6.3 Determining the functional reliability of the gas analysis device based on the measurement results of the measuring.

7. Method according to claim 6, wherein the functional reliability of the gas analysis device is determined based on a comparison of the measurement results of the measurement of the gas flow with predetermined measurement data.

8. Method according to at least one of claims 6 to 7, wherein the gas analysis device is controlled based on the determination of the functional reliability of the gas analysis device.

## Revendications

1. Dispositif d'analyse de gaz, lequel comprend :
1.1 une sonde de prélèvement de gaz (10) pour prélever du gaz à partir d'un volume de gaz (V),
1.2 un analyseur de gaz (50) pour l'analyse du gaz,
1.3 une voie d'écoulement de gaz (70) le long de laquelle un flux de gaz peut être guidé de la sonde de prélèvement de gaz (10) à l'analyseur de gaz (50),
1.4 au moins un dispositif de mesure (80) par lequel le flux de gaz peut être mesuré, et
1.5 une pompe de gaz (40) disposée selon la technique des fluides entre la sonde de prélèvement de gaz (10) et l'analyseur de gaz (50) par laquelle le flux de gaz peut être transporté le long de la voie d'écoulement de gaz (70),
**caractérisé en ce qu'**
1.6 au moins un dispositif de mesure (80) comprend au moins un dispositif de mesure de pression (81), par lequel la pression de gaz peut être mesurée dans la voie d'écoulement de gaz (70) entre la sonde de prélèvement de gaz (10) et la pompe de gaz (40) et comprend au moins un dispositif de mesure de pression (82) par lequel la pression de gaz peut être mesurée dans la voie d'écoulement de gaz (70) entre la pompe de gaz (40) et l'analyseur de gaz (50).

2. Dispositif d'analyse de gaz selon au moins la revendication précédente, sachant que la sonde de prélèvement de gaz (10) comprend un filtre (11).

3. Dispositif d'analyse de gaz selon au moins l'une quelconque des revendications précédentes, sachant que le dispositif d'analyse de gaz (1) comprend un refroidisseur de gaz (30).

4. Dispositif d'analyse de gaz selon l'une quelconque des revendications précédentes, sachant que le dispositif d'analyse de gaz (1) comprend un chauffage (20).

5. Dispositif d'analyse de gaz selon les revendications 3 et 4, sachant que le dispositif de mesure de pression (81) est disposé de telle manière que la pression de gaz peut être mesurée dans la voie d'écoulement de gaz (70) entre le refroidisseur de gaz (30) et la pompe de gaz (40).

6. Procédé de détermination de l'efficacité d'un dispositif d'analyse de gaz, lequel comprend les étapes suivantes :
6.1 mise à disposition d'un dispositif d'analyse de gaz selon au moins l'une quelconque des revendications précédentes,
6.2 mesure d'un flux de gaz s'écoulant le long de la voie d'écoulement de gaz,
6.3 détermination de l'efficacité du dispositif d'analyse de gaz sur la base des résultats des résultats de mesure de la mesure.

7. Procédé selon la revendication 6, pour lequel l'efficacité du dispositif d'analyse de gaz est déterminée sur la base d'une comparaison des résultats de mesure de la mesure du flux de gaz aux données de mesure prédéfinies.

8. Procédé selon au moins l'une quelconque des revendications 6 à 7, pour lequel le dispositif d'analyse de gaz est commandé sur la base de la détermination de l'efficacité du dispositif d'analyse de gaz.
